Europäisches Patentamt

**European Patent Office** (11) Veröffentlichungsnummer: **0 047 465**
**B1**

Office européen des brevets

(12) **EUROPÄISCHE PATENTSCHRIFT**

(45) Veröffentlichungstag der Patentschrift:
24.07.85

(21) Anmeldenummer: 81106801.4

(22) Anmeldetag: 01.09.81

(51) Int. Cl.⁴: **A 61 M 25/00**

(54) **Perkutaner Ballonkatheter.**

(30) Priorität: **10.09.80 US 185762**

(43) Veröffentlichungstag der Anmeldung:
**17.03.82 Patentblatt 82/11**

(45) Bekanntmachung des Hinweises auf die Patenterteilung:
**24.07.85 Patentblatt 85/30**

(84) Benannte Vertragsstaaten:
**CH DE FR GB IT NL SE**

(56) Entgegenhaltungen:
**WO - A - 81/02110**
**DE - A - 2 915 089**
**FR - A - 439 636**

**Prospekt der Firma Datascope "Percor Intra-Aortic Balloon for Percutaneous Insertion"**

(73) Patentinhaber: **Kontron Incorporated, Nine Plymouth Street, Everett Massachusetts 02149 (US)**

(72) Erfinder: **Lombardi, Edward J., 143 Oakland Street, Malden Mass. 02148 (US)**
Erfinder: **Neuman, Harold L., 35 Colonial Drive, Reading, Mass. 01867 (US)**
Erfinder: **Magro, Alfred E., Nine Rich Road, Woburn, Mass. 01801 (US)**
Erfinder: **Rishton, Michael L., 37 Covey Hill Road, Reading, Mass. 01867 (US)**

(74) Vertreter: **Buntz, Gerhard et al, Grenzacherstrasse 124 Postfach 3255, CH-4002 Basel (CH)**

**Beschreibung**

Die Erfindung betrifft einen intra-arteriellen Ballonkatheter mit einem aufblasbaren und entlüftbaren Ballon, der ein distales und ein proximales Ende aufweist, wobei das proximale Ende des Ballons dicht mit dem distalen Ende eines hohlen Katheters verbunden ist, um ein Fluid zum Ballon hin und von diesem weg zu führen, und einem drehbar im Katheter und im Ballon angeordneten langgestreckten Element, dessen distales Ende fest mit dem distalen Ende des Ballons verbunden ist.

Herkömmliche intra-arterielle Ballonkatheter, die auch als Ballonpumpen bezeichnet werden, sind beispielsweise in US-A-3 692 018; US-A-3 504 662 und US-A-3 939 820 beschrieben und gezeigt.

Mit wachsender Erfahrung wurden die ursprünglichen Indikationen für intra-arterielle Ballonpumpen, nämlich Herzversagen aufgrund akuter Infarzierung, nachoperatives, extrem niedriges Herzminutenvolumen und Unmöglichkeit der Entwöhnung von einem pneumocardialen Bypass, erweitert und umfassen hartnäckig instabile Angina in der Periode vor und nach einer Infarzierung, wiederholte lebensbedrohende Tachiarrhytmien und voroperative Unterstützung beim Bestehen einer schweren links-ventrikulären Dysfunktion. Vor kurzem wurden intra-arterielle Ballonpumpen sowohl experimentell als auch klinisch angewendet, um das Infarktausmass zu reduzieren.

Während der ersten Jahre nach der Einführung des intra-arteriellen Ballonkatheters für klinische Anwendung im Jahre 1968, waren diese Gegenstand erheblicher Entwicklungtätigkeit und konstruktiven Aufwands. In den 70er Jahren war der grösste Teil des Aufwands auf dem Gebiet der intra-arteriellen Ballonpumpe hauptsächlich dahingehend gerichtet, die Indikationen für dieses Therapeutikum herauszuarbeiten und über die umfangsreichen klinischen Erfahrungen zu berichten. Während dieser Periode gab es keine grösseren Fortschritte in der Technologie. Nun konzentriert sich die Aufmerksamkeit jedoch wieder auf die Verbesserung der Konstruktion von Ballonkathetern. Ein hauptsächlicher konstruktiver Nachteil des herkömmlichen intra-arteriellen Ballonkatheters besteht darin, dass er durch einen operativen Eingriff in die Arteria femoralis eingeführt werden muss. Es ist klar, dass dies eine erhebliche Verzögerung bei der Anwendung des Geräts mit sich bringt.

Der einzige derzeit bekannte und verfügbare perkutane und intra-arterielle Ballonkatheter ist das mit dem Warenzeichen PERCOR bezeichnete System der Firma Datascope (vgl. Firmenprospekt). Dieser Ballonkatheter enthält einen Einkammerballon, der dicht am Ende des Katheters angeordnet ist. Das äusserste Ende des Ballons ist im Inneren an einem steifen Draht im Ballon befestigt, der in einem Gelenk endet, das sich an der Verbindungsstelle zwischen Katheter und Ballon befindet. Der Ballon wird um den Draht herumgewickelt, indem der Katheter festgehalten wird und das andere Ende des Ballons gedreht wird, bis der gewünschte Grad des Aufwickelns erreicht ist. Nachdem der Ballon aufgewickelt ist, verlässt man sich auf ein an das Innere des Ballons angelegtes Vakuum, um den Ballon im aufgewickelten Zustand zu halten. Während der Einführung ist ein ständiges Drehen angezeigt, um eine Lockerung der Ballonwicklung zu festigen. Weder während, noch nach der Einführung ist der Zugang zum Inneren der Aorta möglich und keine positiven Massnahmen stellen sicher, dass der Ballon während der Einführung vollständig aufgewickelt bleibt, oder dass der Ballon nach der Einführung vollständig auseinandergewickelt ist, bevor der Pumpvorgang beginnt. Die Anwendung eines positiven Drucks im Inneren des Ballons zusammen mit manuellem Rotieren des Katheters, soll sicherstellen, dass der Ballon auseinandergewickelt wird und das Pumpen beginnen kann; ein Wiederaufwickeln des Ballons zur Entfernung des Katheters ist weder möglich noch empfehlenswert. Mit dieser Art Ballonpumpe traten beim Auseinanderwickeln des Ballons und beim Durchgang des Katheters durch die Aorta Fehlfunktionen auf.

Der Erfindung liegt die Aufgabe zugrunde, einen neuen und verbesserten Ballonkatheter bereitzustellen, der perkutan eingeführt werden kann und der kontrolliertes, aufeinanderfolgendes Aufwickeln und Auseinanderwickeln des Ballons erlaubt. Zumindest bei einem Teil der Ausführungsformen des neuen Ballonkatheters sollte die Verwendung eines Führungsdrahts und der Zugang zum Inneren des Blutgefässes, in welcher der Katheter eingeführt ist, möglich sein.

Erfindungsgemäss wird dies erreicht durch eine Vorrichtung der eingangs erwähnten Art, die sich dadurch auszeichnet, dass sich das langgestreckte Element über die Länge des Ballons und des Katheters erstreckt, dass eine Drehbetätigungseinrichtung zum Bewirken des Aufwickelns und Auseinanderwickelns des Ballons um das langgestreckte Element herum vorgesehen ist und dass die Drehbetätigungseinrichtung zwei einander koaxial umfassende, gegeneinander verdrehbare Teile aufweist, von denen der eine Teil fest mit dem proximalen Ende des Katheters und der andere Teil fest mit dem proximalen Ende des langgestreckten Elements verbunden ist.

Eine bevorzugte Ausführungsform der vorliegenden Erfindung enthält einen intra-arteriellen Ballonkatheter mit einem Einkammer-Ballon, der dicht mit dem Ende eines Katheters verbunden ist, und sich für die nicht-operative Einführung nach der standardisierten Seldinger-Technik durch die Haut in die Arteria femoralis eignet. Das äusserste Ende des Ballons ist vorzugsweise fest mit einem Ende eines separaten und vorzugsweise im wesentlichen verwindungssteifen, jedoch in Achsquerrichtung flexiblen Röhrchens verbunden, das ein Drehmoment ohne wesentliche Verformung verträgt. Das Röhrchen ist an beiden Enden offen, befindet sich im Katheter und erstreckt sich über dessen ganze Länge. Der Katheter endet in einer Verzweigung oder Weiche, deren einer Arm das Röhrchen aufnimmt und deren anderer Arm zur Verbindung mit einer herkömmlichen Pumpvorrichtung geeignet ist, um die Pumpfunktion des Ballons durch den Katheter vorzunehmen. Das dem Ballon entgegengesetzte Ende des Röhrchens ist fest mit einer Drehbetätigungseinrichtung verbunden, die an der Verzweigung oder Weiche angebracht ist, und mit der das Röhrchen im Katheter gedreht werden kann, was zu einem kontrollierten, aufeinanderfolgenden Aufwickeln und Auseinanderwickeln des Ballons um das Röhrchen herum führt. Im Betrieb wird der Ballon in herkömmlicher Art und Weise über den Katheter aufgepumpt, und über das Röhrchen besteht ein Zugang zum äussersten Ende des Ballons und zum Inneren der Aorta.

Die vorliegende Erfindung ermöglicht die schnelle und einfache Einführung eines intra-arteriellen Ballon-Katheters durch die Haut unter Verwendung einer geeigneten Katheterisierungstechnik, wie beispielsweise der Seldinger-Technik. Die vorliegende Erfindung erlaubt ausserdem das kontrollierte und aktive Aufwickeln des Ballons vor und während der Einführung, das kontrollierte und aktive Auseinanderwickeln des Ballons, nachdem dieser eingeführt ist, und das aktive und kontrollierte Wiederaufwickeln des Ballons zur Entnahme. Das Vorsehen eines drehbaren Röhrchens im Katheter, das an beiden Enden offen ist, in Kombination mit der Betätigungseinrichtung, die sich am Katheter befindet, erlaubt ausserdem die Verwendung eines Führungsdrahts zur Erleichterung der Einführung, sowie den Zugang zum Inneren der Aorta, nachdem der Ballon eingeführt ist.

Ein weiteres Ausführungsbeispiel der vorliegenden Erfindung, das keinen Zugang zum Inneren der Aorta ermöglicht, sieht einen flexiblen Draht vor, der drehbar im Katheter angeordnet ist und dessen eines Ende im Inneren des Ballons an dessen äusserstes Ende fixiert ist und dessen anderes Ende mit der Betätigungseinrichtung verbunden ist, die an der Verzweigung angebracht ist. Dadurch kann der Draht in kontrollierter Weise im Inneren des Katheters gedreht werden, was wiederum zu einem kontrollierten Aufwickeln und Auseinanderwickeln des Ballons um den Draht herum führt. Die Verwendung eines Drahts ergibt einen minimalen äusseren Durchmesser für den aufgewickelten Ballon und damit einen minimalen inneren Durchmesser des Katheters.

Vorzugsweise wird ein verhältnismässig weiches und flexibles Endteil am Röhrchen oder am Draht vorgesehen, das so weich ist, dass Verletzungen der Aorta durch den Ballon ausgeschlossen sind, die bei Verwendung von hypodermischen Kanülen, Draht usw. über die ganze Länge vorkommen könnten.

Falls das Endteil des Röhrchens aus relativ weichem und flexiblem Schlauch besteht, ist es vorteilhaft, einen Dorn oder Draht zu verwenden, der entfernbar in das Röhrchen eingeführt werden kann und sich über dessen gesamte Länge erstreckt, um ein Abknicken des flexiblen Teils zu verhindern, während der Ballon vor dem Einführen oder Entfernen aufgewickelt wird. Nachdem der Ballon aufgewickelt ist, wird der Dorn nicht länger benötigt und kann entfernt werden. Im Folgenden werden Ausführungsbeispiele der Erfindung unter Bezugnahme auf die beiliegenden Zeichnungen beschrieben. Es zeigen

Fig. 1 einen Längsschnitt durch einen intra-arteriellen Ballonkatheter nach der Erfindung, mit dem Ballon im auseinandergewickelten Zustand;

Fig. 2 eine teilweise geschnittene Ansicht der in Fig. 1 gezeigten Vorrichtung mit dem Ballon in aufgewickeltem Zustand;

Fig. 3 einen vergrösserten Schnitt durch einen Ballon mit einem weichen und flexiblen Endteil des Röhrchens;

Fig. 4 eine Seitenansicht eines Dorns zur Verwendung in einer Ausführungsform mit einem Röhrchen mit flexiblem Endteil;

Fig. 5 einen Längsschnitt durch eine weitere Ausführungsform der vorliegenden Erfindung.

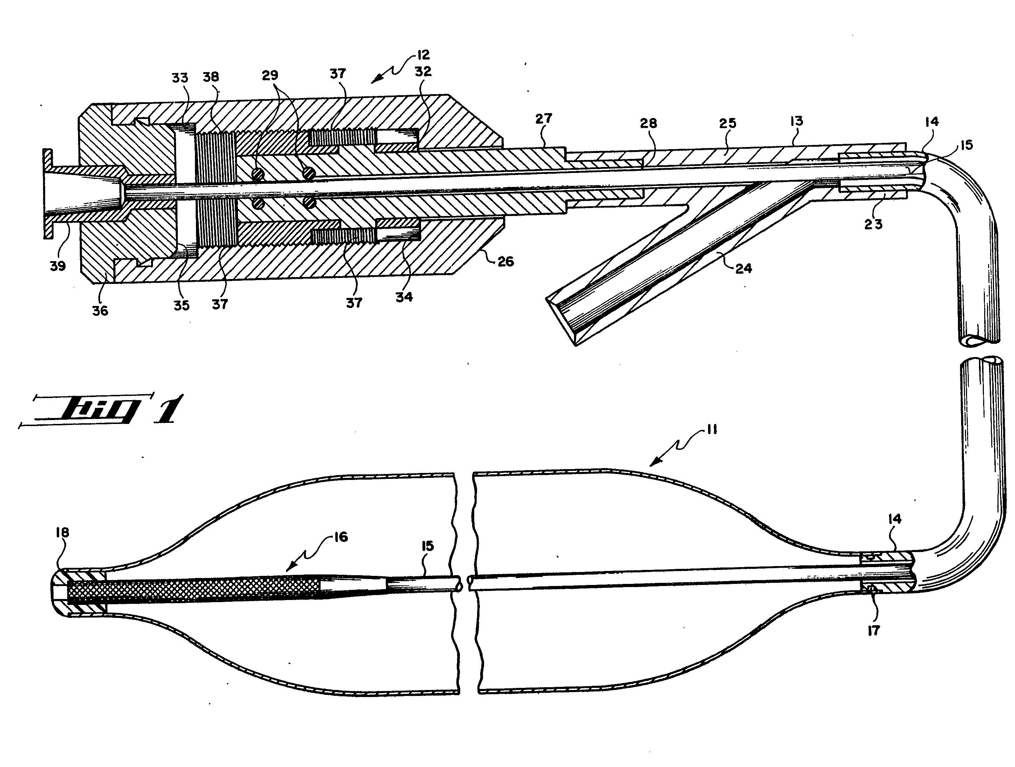

Wie in Fig. 1 gezeigt, enthält ein Ballonkatheter nach der vorliegenden Erfindung einen herkömmlichen intra-arteriellen Einkammer-Ballon 11 und eine Drehbetätigungseinrichtung 12, die durch eine Weiche 13 und einen Katheter 14 miteinander verbunden sind und innerhalb deren sich, wie im Folgenden näher beschrieben, ein Röhrchen 15 befindet, mit dem der Ballon kontrolliert aufgewickelt und auseinandergewickelt werden kann. Der Einkammer-Ballon wird lediglich beispielsweise gezeigt und könnte selbstverständlich auch durch einen Mehrkammer-Ballon ersetzt sein.

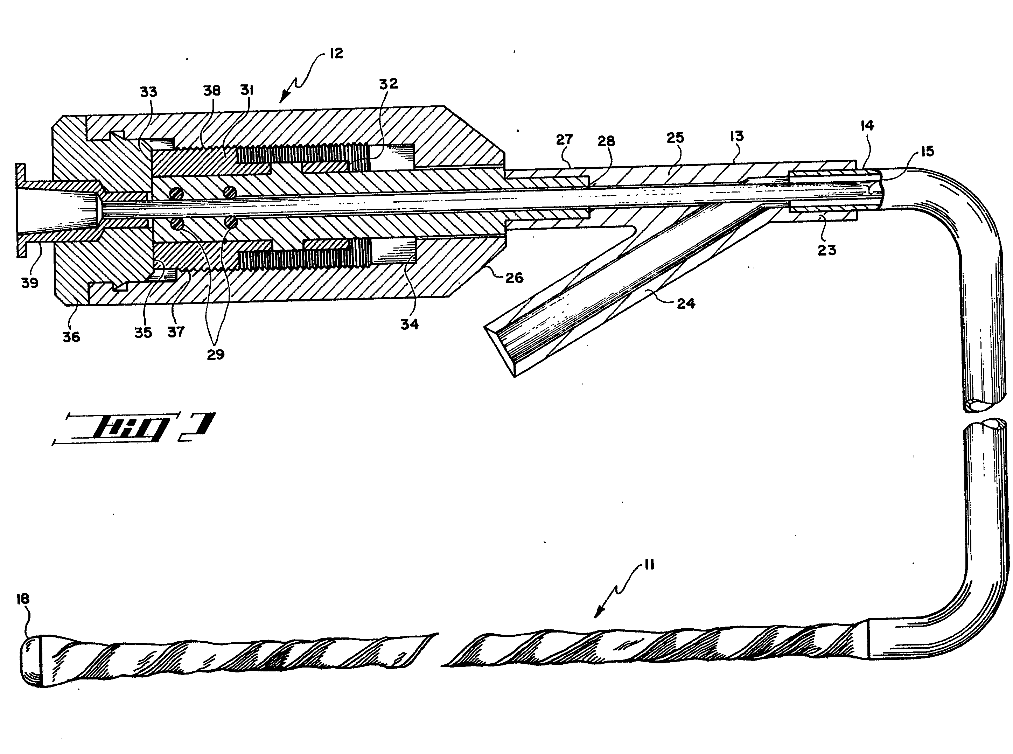

Der Ballon 11 besteht vorzugsweise aus einem antitrombogenen, flexiblen Material und ist mit seinem proximalen Ende dicht mit dem distalen Ende des Katheters 14 und an seinem distalen Ende mit dem flexiblen Endteil 16 des Röhrchens 15 verbunden. Es ist ohne weiteres ersichtlich, dass ein Drehen des Röhrchens 15 relativ zum Katheter 14 zu einem Aufwickeln des Ballons 11 um das in seinem Inneren befindliche Röhrchen 15 herum führt, wie es in Fig. 2 gezeigt ist. Der äussere Durchmesser des Katheters 14 ist an dem Verbindungspunkt mit dem Ballon 11 vorzugsweise reduziert, um einen konstanten äusseren Durchmesser zu erzielen. Zusätzlich kann an dieser Stelle ein Metallring 17 vorgesehen sein, um das proximale Ende des Ballons leichter sichtbar zu machen und damit die Plazierung des Ballons zu erleichtern.

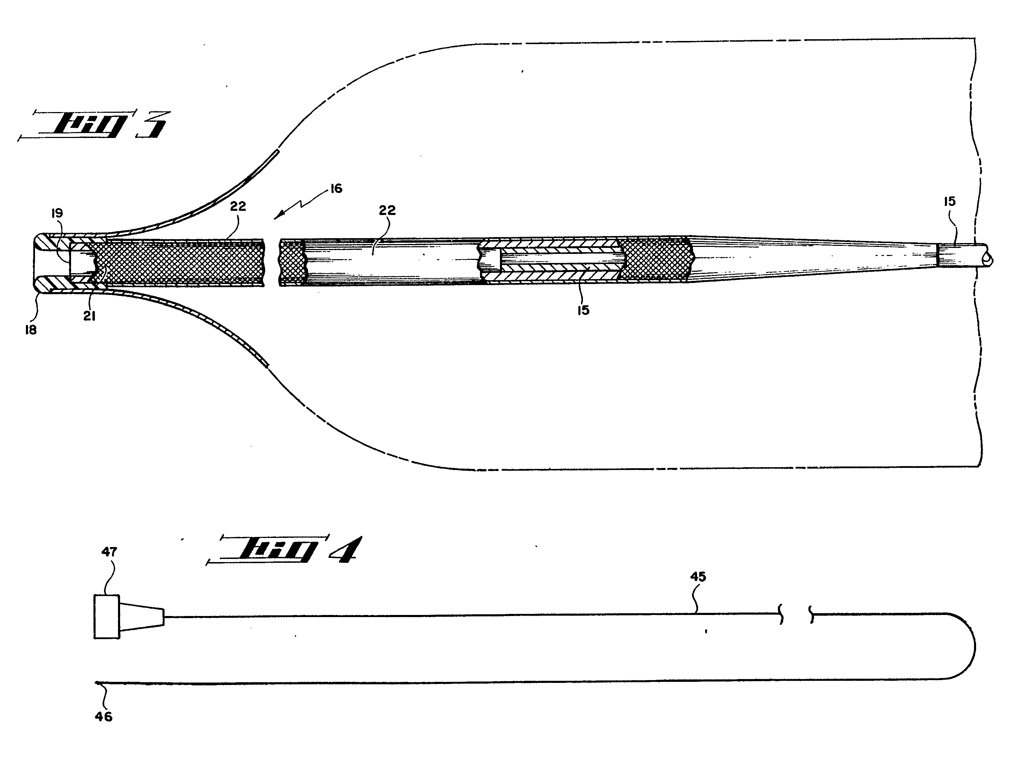

Betrachtet man nun das distale Ende des Ballons in den Fig. 1 und 3, so stellt man fest, dass ein Endteil 16 mit einer vorzugsweise relativ hohen Flexibilität zwischen dem Röhrchen 15 und einer steifen Spitze 18 angeordnet ist, die dicht mit dem distalen Ende des Ballons verbunden ist. Der hintere Teil der Spitze 18 hat einen verringerten äusseren Durchmesser, um die Spitze des Ballons aufzunehmen, und einen vergrösserten Innendurchmesser, um das äussere Ende des flexiblen Endteils 16 aufzunehmen. Das entgegengesetzte Ende des flexiblen Teils 16 ist mit dem äussersten Ende des Röhrchens 15 verbunden. Das Endteil 16 besitzt eine Konstruktion und/oder ein Material, das trotz einer wesentlichen Flexibilität oder Weichheit Drehkräfte aushält, um eine Verdrillung zu vermeiden, wenn der Ballon aufgewickelt wird. Dies wird beispielsweise, wie am besten aus Fig. 3 ersichtlich, dadurch erreicht, dass ein innerer Kunststoffschlauch mit einem Drahtnetz 21 überdeckt ist, welches wiederum mit einem durch Hitzeeinwirkung schrumpfenden Schlauch 22 überzogen ist. Wenn der Schlauch 22 über dem Netz 21 geschrumpft ist, ergibt sich ein flexibles Element, das ein Drehmoment von vernünftiger Höhe, wie es beim Aufwickeln des Ballons auftritt, in zufriedenstellender Weise aushält. Der innere Schlauch 19 und das Netz 21 sind innerhalb der Spitze 18 angeordnet und mit dieser verbunden. Auf der entgegengesetzten Seite ist der innere Schlauch über das Ende des Röhrchens 15 gezogen, ein überstehender Teil des Netzes mit diesem verbunden und mit dem Schrumpfschlauch 22 abgedeckt.

Neben dieser beschriebenen, speziellen Form des flexiblen Endteils sind auch andere Ausführungsformen denkbar, die selbstverständlich, ohne den Rahmen der Erfindung zu verlassen, eingesetzt werden können. Es ist lediglich notwendig, dass das flexible Endteil einen relativ geringen Widerstand gegenüber seitlichen Biegungen, aber einen hohen Widerstand gegenüber Drehmomenten aufweist, um eine Drehverformung zu verhindern.

Für das Röhrchen 15 hat sich hypodermatisches Schlauchmaterial mit einem Innendurchmesser von ca. 1 mm und einem Aussendurchmesser von ca. 1,25 mm als geeignet erwiesen. Wenn dieses in einem Katheter der Grösse F 12 (ungefähr 4 mm) angeordnet ist, kann die perkutane Einführung mit herkömmlicher Katheterisierungseinrichtung erfolgen und Gas zum und vom Ballon frei fliessen.

Das proximale Ende des Katheters 14 ist dicht mit einem ersten Arm 23 der Weiche 13 verbunden, die aus einem geeigneten Kunststoffmaterial besteht, wie beispielsweise einem Urethancopolymer. Ein zweiter Arm 24, der mit dem ersten Arm 23 in Verbindung steht, dient zur Verbindung mit der Konsole, die eine herkömmliche Pumpeinrichtung zum Aufblasen und Entlüften des Ballons durch die Arme 23 und 24 und den Katheter 14 enthält. Das Röhrchen 15 führt frei und drehbar koaxial durch die Arme 23 und 25 der Weiche 13 und erstreckt sich bis zum Handgriff 26, mit dem es fest verbunden ist und der den äusseren Teil der Betätigungseinrichtung 12 darstellt. Im Handgriff 26 befindet sich ein Gewinde- und Wicklungsbegrenzungsteil 27, das fest mit dem Arm 25 der Weiche 13 verbunden ist und eine zentrale Öffnung 28 aufweist, welche das Röhrchen 15 frei beweglich aufnimmt. In der Öffnung 28 befinden sich O-Ringe 29 zur Verhinderung eines Lecks während der Ballon aufgeblasen und entlüftet wird. Da sich bei der Ausführungsform nach Fig. 2 das Röhrchen 15 innerhalb des Begrenzungs- und Gewindeteils 27 dreht, und alle mit dem Inneren des Katheters 14 verbundenen Anschlüsse gasdicht sind, bewirken die O-Ringe 29 eine Dichtung an der einzig möglichen Leckstelle zum bzw. vom Katheter 14. In fester Verbindung am Äusseren des rückwärtigen Teils des Begrenzungs- und Gewindeteils 27, beispielsweise durch ein Spritzgussverfahren geformt, befindet sich ein Aussengewindeteil 31, das einen Auseinanderwicklungsanschlag 32 und einen Aufwicklungsanschlag 33 aufweist. Während das Begrenzungs- und Gewindeteil 27 aus einem Urethancopolymer oder ähnlichem bestehen kann, wird der Aussengewindeteil 31 vorzugsweise aus einem härteren Metarial hergestellt, beispielsweise einem Acetalharz, in welches ein Gewinde geschnitten oder eingegossen ist. Die Anschlagfläche 32 wirkt mit einer inneren Anschlagfläche 34 am Handgriff 26 zusammen, um die Bewegung des Handgriffs 26 zu begrenzen, wenn bis zur vollständig auseinandergewickelten Position gedreht wird, die in Fig. 1 gezeigt ist. Entsprechend wirkt die Fläche 33 mit dem inneren Anschlag 35 am Deckel 36 zusammen, der einen Teil des Handgriffs 26 bildet, um wiederum die Bewegung des Handgriffs 26 zu begrenzen, wenn bis zu der in Fig. 2 gezeigten vollständig aufgewickelten Position gedreht wird. Der Handgriff 26 ist in Fig. 1 in dem Zustand gezeigt, der der vollständig auseinandergewickelten Situation entspricht, und in Fig. 2 in dem Zustand, der dem vollständig aufgewickelten Ballon entspricht. Der Handgriff 26 besitzt ein Innengewinde 37, das in das Aussengewinde 38 des Gewindeteils 31 eingreift.

Die Gewinde des Drehbetätigungsmechanismus haben vorzugsweise einen Durchmesser von etwa 12,5 mm und eine Ganghöhe von etwa 0,4 mm um eine zum festen Aufwickeln des Ballons ausreichende Zahl von Umdrehungen des Röhrchens 15 zu ermöglichen, ohne dasselbe übermässig zu dehnen, während gleichzeitig eine minimale Längsbewegung des Handgriffs 26 nötig ist. 18 Umdrehungen mit einer Längsbewegung von etwa 6,5 mm wurde als zufriedenstellend für einen intra-arteriellen Ballon mit einem Volumen von 40 cm³ gefunden.

Das proximale Ende des Handgriffs 26 ist mit einem Deckel 36 verschlossen, der in das offene Ende des Handgriffs 26 eingepresst ist und mit diesem nach Justierung der Gewinde fest verbunden wird. Die Abstände zwischen den Anschlagflächen 32, 33 und 34, 35 sind so gewählt, dass die gewünschte Zahl von Umdrehungen des Handgriffs für den Ballon, mit dem dieser benutzt wird, möglich ist.

Falls eine grössere oder geringere Zahl von Umdrehungen oder eine grössere oder geringere Längsbewegung gewünscht wird, kann dies einfach und leicht dadurch erreicht werden, dass die Gewindeganghöhe und die Abstände zwischen den Anschlägen entsprechend gewählt werden.

Koaxial angeordnet befindet sich im Deckel 36 eine herkömmliche Luer-Armatur 39, mit der das Röhrchen 15 fest verbunden ist. Die Luer-Armatur 39 ist auch mit dem Deckel 36 fest verbunden, so dass beim Drehen des Handgriffs 26 auch das Röhrchen 15 gedreht wird, und dadurch das kontrollierte aufeinanderfolgende Aufwickeln und Auseinanderwickeln des Ballons erfolgt.

Betrachtet man nun Fig. 2, die die Ausführungsform nach Fig. 1 in vollständig aufgewickeltem Zustand zeigt, so stellt man fest, dass der Durchmesser des Ballons in seinem aufgewickelten Zustand nicht grösser, sondern etwas kleiner ist als der des Katheters 14. Dieser Umstand erleichtert die Einführung und die Entfernung des Ballons durch den nicht gezeigten Einführungskatheter, der bei der sogenannten Seldinger-Technik üblicherweise benutzt wird.

Das Röhrchen 15 und der flexible Teil 16 werden bezüglich ihrer Länge so gewählt, dass der Ballon seinen normal voll aufgeblasenen Zustand erreicht, ohne belastet oder gedehnt zu werden. Die vorstehend beschriebene Dreheinrichtung erlaubt lediglich das korrekte Mass des Aufwickelns und Auseinanderwickelns mit in jedem Falle unbedeutender Ausdehnung des Ballons durch die Aufwicklung.

Selbstverständlich sind auch Modifikationen für die Dreheineinrichtung denkbar. So ist es beispielsweise nicht nötig, dass die Drehung der Betätigungseinrichtung 12 eine Längsbewegung des Röhrchens 15 während des Aufwickel- oder Entwickelvorgangs durchführt. Es ist auch nicht erforderlich, dass Dichtungen, wie beispielsweise die O-Ringe 29 verwendet oder so plaziert werden, wie es vorstehend gezeigt ist, um ein Leck zu verhindern. Eine weitere Modifikation besteht darin, dass die Weiche 13 direkt einen Teil des Katheters oder der Drehbetätigungseinrichtung 12 darstellt, oder dass sie überhaupt fehlt. Falls die Weiche 13 fehlt, ist ersatzweise ein anderer Zugang zum Inneren des Katheters vorzusehen.

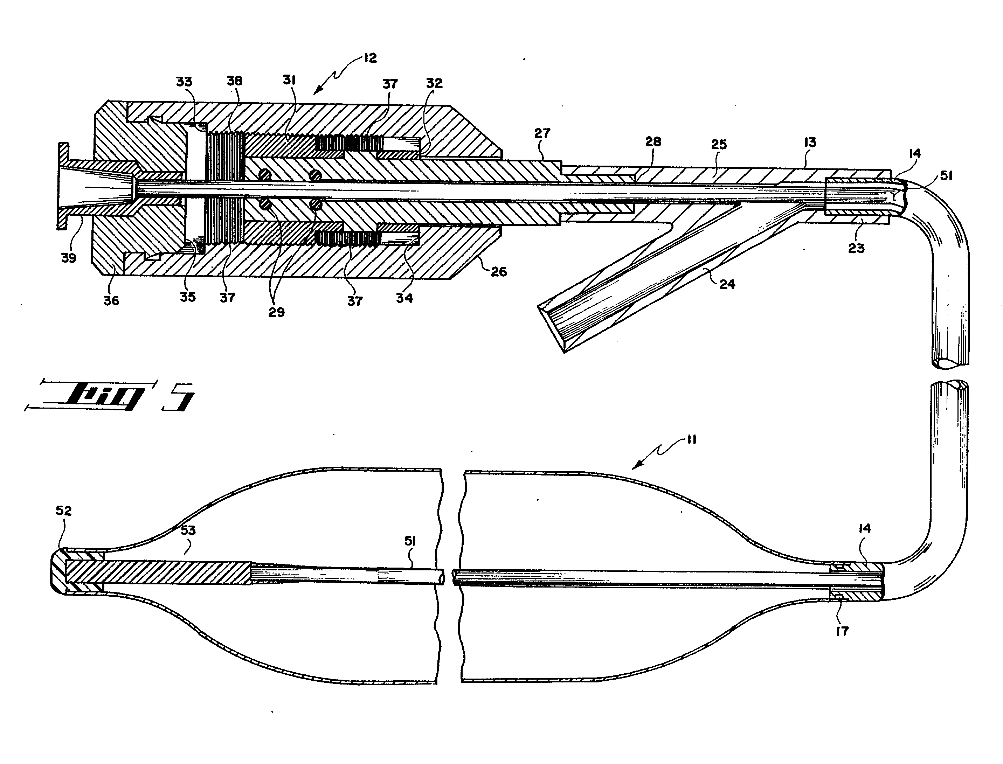

Fig. 4 zeigt einen Dorn oder Draht 45 zur Einführung in das Röhrchen 15 vor der Aufwicklung des Ballons, wenn das Röhrchen 15 mit einem flexiblen Teil endet, der einen Teil oder die ganze Länge des Ballons ausmacht. Es wurde gefunden, dass in diesem Fall beim Aufwickeln des Ballons ohne einen Dorn 45 der flexible Teil dazu neigt, sich zu verziehen und dass dadurch das Aufwickeln des Ballons beeinträchtigt wird. Die Verwendung des Dorns 45 stellt sicher, dass der Ballon gleichmässig aufgewickelt wird.

Der Dorn 45 ist so lang, dass sein distales Ende 46 in die Spitze 18 reicht. Das proximale Ende des Dorns 45 endet in einer Armatur 47, die in die Armatur 39 im Deckel 36 passt. Nachdem der Ballon aufgewickelt ist, wird der Dorn 45 nicht länger benötigt und kann entfernt werden.

Fig. 5 zeigt eine andere Ausführungsform der Erfindung, bei der im Unterschied zu der in Fig. 1 gezeigten Ausführungsform das Röhrchen 15 durch einen Draht 51 ersetzt ist.

Der Draht 51 ist an einem Ende fest mit der Luer-Armatur 39 verbunden und erstreckt sich durch entsprechend dimensionierte O-Ringe 29, Begrenzungs- und Gewindeteil 27, Weiche 13, Katheter 14 und Ballon 11. Das distale Ende des Drahts 51 ist fest mit einem flexiblen Endteil 53 verbunden, dessen distales Ende fest mit der Spitze 52 verbunden ist, wodurch der Ballon in gleicher Weise nacheinander aufgewickelt und auseinandergewickelt werden kann. wie dies bei der Ausführungsform nach Fig. 1 beschrieben ist.

Wie im Falle von hypodermatischen Kanülen und dergleichen, ist der Draht 51 verhältnismässig steif. Wenn ein derartiges Material oder ein Draht bis in die Spitze des Ballons reicht, so ergeben sich Schwierigkeiten beim Einführen des Katheters in einem teilweise verlegten oder stark gekrümmten Blutgefäss. Trotzdem ist es, wenn auch weniger wünschenswert, möglich, das in den Fig. 1, 2, 3 und 5 gezeigte flexible Endteil wegzulassen, wobei allerdings die Verletzungsgefahr und/oder Schwierigkeiten bei der Einführung des Katheters vergrössert werden.

Während die Ausführungsform nach Fig. 5 das kontrollierte, aufeinanderfolgende Aufwickeln und Auseinanderwickeln des Ballons erlaubt, lässt sie jedoch die Verwendung eines Führungsdrahts zur Einführung des Ballons oder den Zugang zum Inneren des Blutgefässes, in den es eingeführt ist, nicht zu. Die Verwendung des Drahts 51 ermöglicht jedoch eine Reduktion des Durchmessers der verschiedenen Komponenten, durch die der Ballon geführt wird.

**Patentansprüche**

1. Intra-arterieller Ballonkatheter mit einem aufblasbaren und entlüftbaren Ballon (11), der ein distales und ein proximales Ende aufweist, wobei das proximale Ende des Ballons dicht mit dem distalen Ende eines hohlen Katheters (14) verbunden ist, um ein Fluid zum Ballon hin und von diesem weg zu führen, und einem drehbar im Katheter (14) und im Ballon (11) angeordneten langgestreckten Element (15, 51) dessen distales Ende fest mit dem distalen Ende des Ballons verbunden ist, dadurch gekennzeichnet, dass sich das langgestreckte Element (15, 51) über die Länge des Ballons (11) und des Katheters (14) erstreckt, dass eine Drehbetätigungseinrichtung (12) zum Bewirken des Aufwickelns und Auseinanderwickelns des Ballons (11) um das langgestreckte Element (15, 51) herum vorgesehen ist und dass die Drehbetätigungseinrichtung (12) zwei einander koaxial umfassende, gegeneinander verdrehbare Teile (26, 27) aufweist, von denen der eine Teil (27) fest mit dem proximalen Ende des Katheters und der andere Teil (26) fest mit dem proximalen Ende des langgestreckten Elements verbunden ist.

2. Ballonkatheter nach Anspruch 1, dadurch gekennzeichnet, dass das langgestreckte Element (15) an seinem distalen Ende ein Endstück (16) mit einer verhältnismässig geringen seitlichen Steifigkeit aufweist.

3. Ballonkatheter nach Anspruch 2, dadurch gekennzeichnet, dass das langgestreckte Element (15) hohl ist.

4. Ballonkatheter nach Anspruch 1, dadurch gekennzeichnet, dass das langgestreckte Element (51) massiv ist.

5. Ballonkatheter nach Anspruch 3, dadurch gekennzeichnet, dass das langgestreckte Element (15) und das Endstück (16) einen offenen Durchgang von und durch die Drehbetätigungseinrichtung (12) zum und durch den Ballon bilden.

6. Ballonkatheter nach Anspruch 5, dadurch gekennzeichnet, dass das langgestreckte Element (15) ein flexibles Metallrohr ist und das Endstück (16) aus einem flexiblen nicht metallischen Schlauch besteht, der fest an dem Metallrohr angebracht ist, wobei das Endteil verwindungssteif ist, um ein Drehmoment ohne wesentliche Verformung aufzunehmen.

7. Ballonkatheter nach Anspruch 1, gekennzeichnet durch einen zusätzlichen, mit dem Inneren des Kathethers in Verbindung befindlichen Eingang (24), wobei die Drehbetätigungseinrichtung (12) Dichtungsmittel (29) zur Verhinderung eines Lecks vom Inneren des Katheters enthält.

8. Ballonkatheter nach Anspruch 7, dadurch gekennzeichnet, dass die Drehbetätigungseinrichtung (12) Mittel (32, 33, 34, 35) zur Begrenzung der Zahl der Umdrehungen enthält.

9. Ballonkatheter nach einem der Ansprüche 1 oder 8, gekennzeichnet durch eine Verzweigung oder Weiche (13) zwischen dem Katheter und der Drehbetätigungseinrichtung, wobei die Weiche zwei koaxiale Arme (23, 25) hat, durch den das langgestreckte Element (15, 51) verläuft, und einen weiteren Arm (24), der die Verbindung mit dem Inneren des Katheters vorsieht.

## Claims

1. An intra-arterial balloon catheter comprising an inflatable and deflatable balloon (11) having a distal end and a proximal end, the proximal end of the balloon being connected in sealing-tight relationship to the distal end of a hollow catheter (14) to convey a fluid to and away from the balloon, an elongate element (15) which is disposed rotatably in the catheter and in the balloon and the distal end of which is secured to the distal end of the balloon, characterised in that the elongate element (15) extends over the length of the balloon (11) and of the catheter (14), a rotary control (12) is provided to roll up and unroll the balloon around the elongate element, and the rotary control comprises two parts (26, 27) which coaxially enclose one another and which are rotatable in relation to one another, one part (27) being secured to the proximal end of the catheter while the other part (26) is secured to the elongate element.

2. A balloon catheter according to claim 1, characterised in that the elongate element (15) has at its distal end an end part (16) having a relatively low lateral rigidity.

3. A balloon catheter according to claim 2, characterised in that the elongate element (15) is hollow.

4. A balloon catheter according to claim 1, characterised in that the elongate element (15) is solid.

5. A balloon catheter according to claim 3, characterised in that the elongate element (15) and the end part (16) form an open passage from and through the rotary control (12) to and through the balloon.

6. A balloon catheter according to claim 5, characterised in that the elongate element (15) is a flexible metal tube and the end part (16) consists of a flexible non-metallic hose which is secured to the metal tube, the end part being torsionally rigid in order to take a torque without any appreciable deformation.

7. A balloon catheter according to claim 1, characterised by an additional inlet (24) communicating with the interior of the catheter, the rotary control (12) containing sealing means (29) to prevent leakage from inside the catheter.

8. A balloon catheter according to claim 7, characterised in that the rotary control (12) contains means (32, 33, 34, 35) for limiting the number of revolutions.

9. A balloon catheter according to claim 1 or claim 8, characterised by a branch or switch (13) between the catheter and the rotary control, the switch having two coaxial arms (23, 25) through which the elongate element (15) extends and another arm (24) which provides the connection to the interior of the catheter.

## Revendications

1. Cathéter intra-artériel à ballon comportant un ballon (11) pouvant être gonflé et désaéré et qui possède une extrémité distale et une extrémité proximale, l'extrémité proximale du ballon étant reliée de façon étanche à l'extrémité distale d'un cathéter creux (14) afin d'envoyer un fluide au ballon et de le retirer de ce dernier, un élément allongé (15) monté rotatif dans le cathéter et dans le ballon et dont l'extrémité distale est reliée rigidement à l'extrémité distale du ballon, caractérisé en ce que l'élément allongé (15) s'étend sur la longueur du ballon (11) et du cathéter (14), qu'il est prévu un dispositif (12) d'entraînement en rotation servant à réaliser l'enroulement et le désenroulement du ballon autour de l'élément allongé, et que le dispositif d'entraînement en rotation comporte deux parties (26, 27) coaxiales l'une dans l'autre et pouvant tourner l'une par rapport à l'autre et dont l'une (27) est reliée rigidement à l'extrémité proximale du cathéter, et dont l'autre (26) est reliée rigidement à l'élément allongé.

2. Cathéter à ballon selon la revendication 1, caractérisé en ce que l'élément allongé (15) comporte, sur son extrémité distale, un embout d'extrémité (16) possédant une rigidité latérale relativement faible.

3. Cathéter à ballon selon la revendication 2, caractérisé en ce que l'élément allongé (15) est creux.

4. Cathéter à ballon selon la revendication 1, caractérisé en ce que l'élément allongé (15) est massif.

5. Cathéter à ballon selon la revendication 3, caractérisé en ce que l'élément allongé (15) et l'embout d'extrémité (16) forment un passage ouvert s'étendant depuis et à travers le dispositif d'entraînement en rotation (12) jusqu'au et à travers le ballon.

6. Cathéter à ballon selon la revendication 5, caractérisé en ce que l'élément allongé (15) est un tube métallique souple et que l'embout d'extrémité (16) est constitué par un tuyau non métallique souple, qui est monté rigidement sur le tube métallique, la partie d'extrémité résistant à la torsion afin d'absorber un couple de rotation sans déformation sensible.

7. Cathéter à ballon selon la revendication 1, caractérisé par une entrée supplémentaire (24) reliée à l'intérieur du cathéter, le dispositif d'entraînement en rotation (12) contenant des moyens d'étanchéité (29) servant à empêcher une fuite à partir de l'intérieur du cathéter.

8. Cathéter à ballon selon la revendication 7, caractérisé en ce que le dispositif d'entraînement en rotation (12) comporte des moyens (32, 33, 34, 35) servant à limiter le nombre des rotations.

9. Cathéter à ballon selon l'une des revendications 1 ou 8, caractérisé par une ramification ou un aiguillage (13) entre le cathéter et le dispositif d'entraînement en rotation, l'aiguillage comportant deux branches coaxiales (23, 25), à travers lesquelles s'étend l'élément allongé (15), et un autre bras (24) qui établit la liaison avec l'intérieur du cathéter.

Fig 1

0 047 465

FIG. 2

Fig 3

18 19 21 22 16 22 15 15

Fig 4

47 45 46

0 047 465

Fig 5

0 047 465